# EUROPEAN PATENT APPLICATION

(11) **EP 3 412 148 A1**
(43) Date of publication of application: **12.12.2018**
(21) Application number: 17382339.4
(22) Date of filing: 06.06.2017
(51) Int. Cl.: A01N 1/02

(54) **SYSTEM AND METHOD FOR CONTROLLED TRANSPORT OF BIOLOGICAL SAMPLES**

(71) Applicant: General Courier Vallès, SL, 08401 Granollers (ES)
(72) Inventor: SÁNCHEZ GIRONELL, Marcel, 08188 Vallromanes (ES); SÁNCHEZ FERNÁNDEZ, Ariadna, 08188 Vallromanes (ES)
(74) Representative: Juncosa Miró, Jaime

(57) **Abstract**

A system and method for the controlled transport of biological samples are disclosed, wherein the system comprises a container including a main body, a cover, an opening/closure control device, a temperature sensor, an electronic board configured for collecting data acquired by said temperature sensor and for controlling a state and operation of said opening/closure control device, and a biological sample content detector controlled by said electronic board. The system further comprises a server including a database; and the electronic board includes a communication module configured for sending to said server, during transport, a series of data at least about the content of the container and the internal temperature thereof, for the storage of said series of data in the database of the server. The biological sample content detector may be a proximity sensor configured for detecting the presence of the biological samples inside the container.

## Description

### Technical field

The present invention generally relates to systems for transporting biological samples. Particularly, the invention relates to a system and a method for the controlled transport of biological samples, for example vaccines or blood, preferably to a medical/health center.

### Background of the Invention

Utility model CN-U-203788986 discloses an insulated transportable cooler for biological samples including a battery, a refrigeration device, an electronic control device, a screen, a door with an electrically controlled lock and a temperature sensor, in addition to wheels and a handle.

Utility model CN-U-202728908 discloses another insulated transportable cooler for biological samples including a battery, an electronic refrigerating sheet, an electronic control device, a screen and a temperature and humidity sensor. It also proposes to include a real time information transmission system.

Patent EP-B1-2341884 discloses a leak-tight bag for biological samples provided with a power supply source, a temperature sensor, a clock, and a system for downloading information, which allows controlling transport conditions and time.

Patent EP-B1-2190750 discloses a container for the metered dispensing of medication including a plurality of compartments intended for containing doses of medication, each provided with a sensor which allows detecting the presence of content therein, the container including an electronic control device, a battery, a screen, and a system for downloading information.

Finally, patent application EP-A1-2436361 discloses a container for transporting biological samples or medical products provided with a cover integrating an electronic control device, a screen, control buttons, a connector for downloading information, sensors, a battery, and a motor driving a cam which causes the cover to be locked or released with respect to the container. The mentioned sensor can be a temperature sensor. Likewise, the authorized users can open or close the container by means of introducing a code through the control buttons, said code being able to be obtained by means of the mentioned information download.

There is however the need for new systems and methods for the controlled transport of biological samples, for example blood, vaccines, infectious substances, among others, by means of a container which allow knowing at all times during transport the conditions of the biological samples and which in turn allow control/supervision of the monitored data of the container, assuring that any handling of the content of the container is recorded.

### Description of the Invention

In a first aspect, the present invention provides a system for the controlled transport of biological samples, which comprises like in the state of the art a container including a main body defining a housing cavity; a cover; an opening/closure control device for controlling the opening/closure of the container; a temperature sensor (more than one can be included) configured for providing information about the internal temperature of the container from an instant of loading the biological samples, for example blood or vaccines, among others, in the container to a subsequent instant of opening the container and/or extracting the biological samples from the container; and an electronic board configured for collecting data acquired by the mentioned temperature sensor and for controlling the state and the operation of the opening/closure control device.

Unlike known proposals, the proposed system further comprises a server with one or more processors and at least one database, or memory. The server is preferably located in a remote area with respect to the container, so the communication module sends the data to the server by means of a wireless connection. The server can be a physical server, a local server, or a cloud server.

Likewise, the container includes a biological sample content detector (more than one can be included) which is controlled by the mentioned electronic board. The electronic board further includes a communication module, preferably a GSM module, configured for sending at least a series of data about the temperature (of the inside of the container) and the content of the container, during transport, to the server for storage of the data in the database, or memory.

The system also preferably includes a communication device such as a mobile telephone or a tablet that is located, in operative situation, in the proximity of the container, and comprising positioning means (for example, a GPS positioning module) operatively connected to the server to allow tracking positioning data of the container during transport.

In one embodiment, the biological sample content detector is a proximity sensor, for example a capacitive, inductive or photoelectric sensor, such as an infrared sensor, configured for detecting the presence of the biological samples transported inside the container from the instant of loading the biological samples in to the instant of extracting the biological samples from the container.

It is particularly important for the proposed system to assure detection of this extraction of the biological samples, extracting only a part of the biological samples from the container thereby being prevented.

The opening/closure control device is preferably included in the cover of the container.

In one embodiment, the opening/closure control device comprises an electromechanical device configured, together with the mentioned electronic board, for being activated preferably by means of two RFID sensors depending on the type of biological samples to be transported.

By means of using the two RFID sensors, the user who opens/closes/handles the container is detected. Likewise, the two RFID sensors allow detecting the name of the center of origin and destination of the biological samples.

The container further comprises one or more batteries for self-powering of the electronic board.

The container also preferably includes a display unit. The display unit can comprise a screen, for example a TFT screen, and/or light-emitting indicators, for examples LEDs.

If the display unit is a screen, in addition to showing discrete information about the content related with the transported biological samples, i.e., whether the container is full or empty, it can also show information relating to the state of the battery/batteries, the temperature inside the container, the user/operator transporting the container, the type of transported sample, the center where the samples were collected, the center where the samples will be transported, etc. If the display unit is LEDs, information about the content of the container can be shown by means of LEDs of different colors.

Likewise, the container can comprise an accelerometer configured for detecting sudden movements of the container during transport or handling.

Other embodiments of the invention disclosed in the present document also include a method for the controlled transport of biological samples in a container, such as the one described in the first aspect of the invention, the method comprising the following steps: detecting, by means of a content detector controlled by an electronic board of the container, the existence of a biological sample load in the container; sending to a server, by means of a communication module included in the electronic board, a series of data about the internal temperature of the container, the content and incidences of opening/closing the container and/or incidences related with content handling; and storing said series of received data and time instants of said incidences of opening/closing the container in a database of the server, wherein the data stored in the database constitutes a control and/or supervision record.

In one embodiment, the method comprises emitting a warning signal if the content detector detects content inside the container once the biological samples have been extracted therefrom.

In another embodiment, the method comprises emitting a warning signal if the temperature of the container is the same as or exceeds a pre-established threshold temperature value during transport.

In yet another embodiment, the method tracks positioning data of the container from an instant of closing to an instant of opening the container, during transport, using positioning means of a computing device (for example the mobile telephone of the operator transporting the container) operatively connected to the server and located in the proximity of the container.

### Brief Description of the Drawings

The foregoing and other advantages and features will be more clearly understood based on the following detailed description of several illustrative and non-limiting embodiments in reference to the attached drawings, in which:
Fig. 1 schematically shows an embodiment of a system for the controlled transport of biological samples.
Fig. 2 shows different views of an embodiment of the container for transporting biological samples proposed by the present invention.
Fig. 3 shows an embodiment of the display unit of the container of Fig. 2.
Fig. 4 is a flow chart describing in detail an embodiment of a method for the controlled transport of biological samples in a container according to this invention.

### Detailed Description of several Embodiments

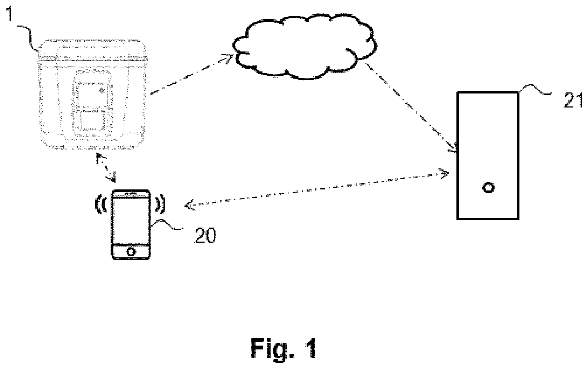

Fig. 1 illustrates an embodiment of a system for the controlled transport of biological samples. According to this embodiment, the system comprises a container 1 for storing and transporting biological samples, for example blood samples, vaccines, among others, including a communication module; a server 21 operatively connected to the mentioned container 1 through a communications network such as the Internet; and a computing device 20 such as a mobile telephone, preferably belonging to the operator (not illustrated for simplicity of the drawing) in charge of transporting the biological samples. The positioning means, for example GPS, integrated in the computing device 20 itself can therefore be used to allow tracking the positioning data of the container 1 in the transportation route.

Alternatively, the server 21 could be a cloud server.

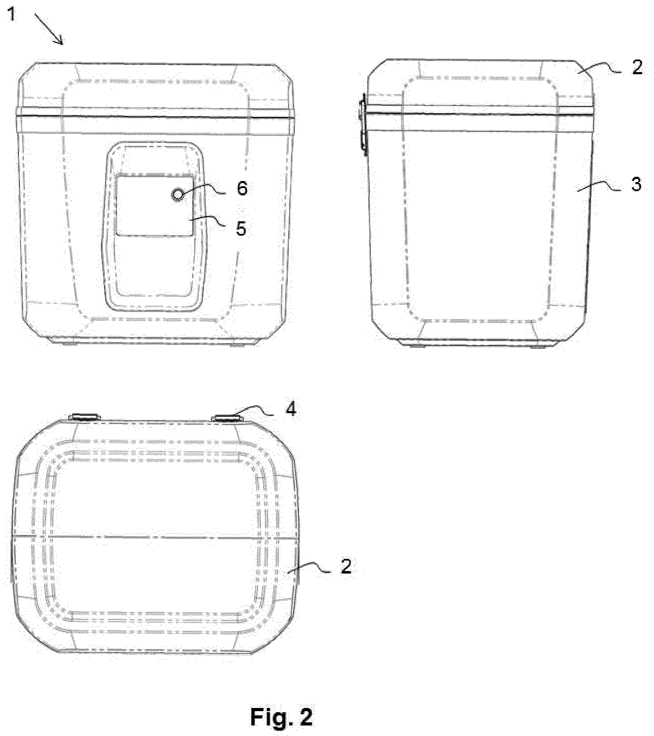

Fig. 2 illustrates an embodiment of the container 1 proposed by the present invention. The container 1 consists of a rigid outer body (or main body) 3 manufactured in plastic materials with an inner foam body (preferably 4 cm thick) conferring it with insulating capacity for keeping the cold. The container 1 has an upper cover 2 fixed with hinges 4. The cover 2 is assembled to the main body 3 of the container 1, generating a leak-tight inner space. To assure leak-tightness, there is a plastic profile both in the upper part of the main body 3 and in the lower part of the cover 2 fitting with one another and incorporating a rubber profile which, when the cover 2 is closed, act as a sealing gasket and make it leak-tight. The container 1 can also include metal hooks on the sides of the main body 3 for holding a strap making it easier to transport the container 1. Likewise, the main body 3 can include on one of its inner sides an elastic band held by rivets fixed at each end which allows holding a folder containing information about the biological samples transported in the container 1.

The inside of the container 1 is lined with a white rigid body of the same material as the outside thereof in order to make it leak-tight and to convert it into a leak-tight space. Preferably, the inner area is sized for storing four racks of biological samples housed inside a secondary container manufactured preferably in a plastic, transparent material, with an opaque cover, in addition to another secondary container having the same or less height for storing different biological samples. The inner area also includes a temperature sensor (not illustrated). The temperature sensor, more than one can be included, provides information about the internal temperature of the container 1 from an instant of loading the biological samples in the container 1 to the instant of opening the container 1 and/or extracting the biological samples from the container 1. The inner area can also include a humidity sensor for providing information about the humidity inside the container 1.

The container 1 also includes an electronic board and a content detector (also not illustrated). The electronic board incorporates the mentioned communication module, for example a GSM or UMTS module, for establishing the mentioned communication with the server 21, sending to said server 21 at least the data about the temperature acquired by the temperature sensor and by the content detector for the storage thereof in its database, or memory.

Likewise, the mentioned cover 2 of the container 1 includes, also not illustrated, an opening/closure control device for controlling the opening/closure of the container 1. The opening/closure control device preferably comprises an electromechanical device configured, together with the mentioned electronic board, for being activated by means of at least two RFID sensors depending on the type of biological samples to be transported. In other words, if the biological samples to be transported are blood samples a first RFID sensor will be used and if the biological samples to be transported are vaccines a second RFID sensor will be used. The cover 2 can also include hooks holding a net which allows holding a series of cold packs, depending on the needs of each case.

The electronic board comprises one or more microprocessors for operating/controlling the different peripherals/elements of the container 1: display unit 5, RFID identification system for identifying the type of biological samples and the users/operators, communication module, content detector, opening/closure control device, temperature sensor, as well as for accommodating supply voltages for the different peripherals/elements, etc.

The content detector is preferably a photoelectric proximity sensor that works by means of infrared. Nevertheless, other proximity sensors can likewise be used for said functionality, for example capacitive or inductive sensors.

The container 1 further includes one or more batteries for self-powering of the electronic board.

In the embodiment of Fig. 2, the display unit 5 comprises a screen, for example a TFT screen. The display unit can be tilted to thereby allow viewing same from different angles. The display unit 5 incorporates a push-button 6 activating the light of the screen 5 at the user's/operator's demand for consulting the data appearing thereon.

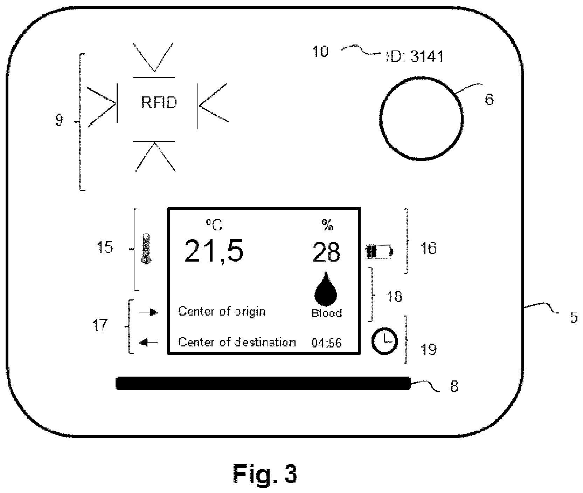

Fig. 3 shows an embodiment of the mentioned screen. As can be seen in Fig. 3, the screen shows data 15 relating to the internal temperature of the container 1, to the remaining percentage of battery 16, to the medical/health center of origin of the biological samples and the center of destination of the biological samples 17, to the type of samples 18 (in this particular case, blood samples, as seen in the text and in the symbol of a drop of blood, it must be pointed out that for other types of samples other symbols will be used (for example a cross to indicate vaccines)) and to the time elapsed during transport 19 (from the closure of the container to the opening thereof in the center of destination). The upper area of the screen includes the reading area of the RFID sensors 9, the identification number 10 of the container 1 and the mentioned push-button 6. The lower area of the screen includes a LED 8 which changes color depending on whether the container is full or empty (preferably, red if the container 1 is full and green if it is empty).

The mentioned screen 5 can also be automatically activated, i.e., the screen 5 can be configured to light up for specific time period.

In an alternative embodiment, the container 1 can include two optoelectronic components, such as LEDs of different colors, to distinctly indicate whether the container 1 is full or empty.

Optionally, the container 1 can include an accelerometer for detecting sudden movements of the container 1 during transport or handling.

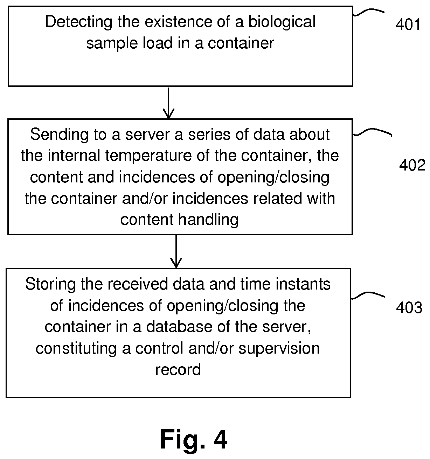

Now, in reference to Fig. 4, said figure shows an embodiment of a method for the controlled transport of biological samples in a container, such as the one described above. The method comprises first detecting, step 401, by means of the mentioned content detector, the existence of a biological sample load in the container 1. Then, the method comprises sending to the server 21, step 402, by means of the mentioned communication module included in the electronic board of the container 1, every certain configurable period of time, a series of data at least relating to: the internal temperature of the container 1, the content and incidences of opening/closing the container 1. Finally, the method stores, step 403, the series of received data and the time instants of said incidences of opening/closing the container 1 in a database of the server 21, wherein the data stored in the database constitutes a control and/or supervision record.

Likewise, the mentioned communication module can also send information relating to the state of the battery/batteries, the medical/health center of origin of the biological samples, the route taken, the identifier of the container 1, the user/operator identifier, in addition to other information memorized in the RFID sensors that may be personalized as required, for example, incidences occurring during transport, such as the wait time, if the container 1 has broken, if an accident has occurred in the route taken, the traffic condition in the route taken, etc.

In one embodiment, the method emits a warning signal if the content detector detects content inside the container 1 once the biological samples have been extracted therefrom. It is thereby assured that all the transported biological samples are extracted in the medical/health center of destination.

Furthermore, if the internal temperature of the container 1 is the same as or exceeds a pre-established threshold temperature value during transport, for example 23 degrees for blood samples or 8 degrees for vaccines, a warning signal can be emitted, said emitted warning signal being recorded in the server 21.

The method allows tracking positioning data of the container 1 from the instant of closing to the instant of opening same, during transport. The mentioned positioning means of the computing device 20 are preferably used for such purpose. Nevertheless, a positioning sensor could be alternatively included in the container 1. The container 1 itself, without having to use the computing device 20, will thereby allow tracking the positioning data of the container 1 during transport.

As improvements of the present invention, the present invention can also provide a mobile application, or an APP, run in the computing device 20 or in another computing device, providing through a user interface the same information as the data stored in the mentioned database (internal temperature of the container, content, incidences, state of the battery/batteries, etc.).

Likewise, the container 1 complies with UN 3373 guidelines in force on the date of filing of this document.

The scope of the present invention is defined in the attached claims.

## Claims

1. A system for the controlled transport of biological samples, comprises a container (1) for storing biological samples including:
- a main body (3) defining a housing cavity;
- a cover (2);
- an opening/closure control device for controlling the opening/closure of the container (1);
- at least one temperature sensor configured for providing information about the internal temperature of the container (1) from an instant of loading said biological samples in the container (1) to a subsequent instant of opening the container (1) and/or extracting the biological samples from the container (1); and
- an electronic board configured for collecting data acquired by said at least one temperature sensor, and for controlling a state and operation of said opening/closure control device;
**characterized in that:**
- the system further comprises a server (21) including a database;
- said container (1) further includes a biological sample content detector controlled by said electronic board; and **in that**
- the electronic board includes a communication module configured for sending to said server (21), during transport, a series of data at least about the content of the container (1) and the internal temperature thereof, for the storage of said series of data in the database of the server (21),
such that the system assures that any handling of the content of the container (1) is recorded.

2. The system according to claim 1, wherein said biological sample content detector is a proximity sensor configured for detecting the presence of the biological samples transported inside the container (1) from the instant of loading the biological samples in to the instant of extracting the biological samples from the container (1).

3. The system according to any one of the preceding claims, wherein said communication module sends the data to the server (21) by means of a wireless connection.

4. The system according to any one of the preceding claims, wherein the opening/closure control device is included in the cover (2).

5. The system according to any one of the preceding claims, wherein the opening/closure control device comprises an electromechanical device configured, together with said electronic board, for being activated, during opening or closure, by means of at least two radio-frequency identification RFID sensors depending on the type of biological samples to be transported.

6. The system according to any one of the preceding claims, further comprising a display unit (5) controlled by the electronic board for indicating distinct information about the content of the container (1) that is at least related with the transported biological samples.

7. The system according to claim 6, wherein said display unit comprises a screen and/or light-emitting indicators.

8. The system according to any one of the preceding claims, wherein the container (1) further comprises one or more batteries for self-powering of the electronic board.

9. The system according to any one of the preceding claims, wherein the container (1) further comprises at least one accelerometer configured for detecting sudden movements of the container (1) during transport or handling.

10. The system according to any one of the preceding claims, further comprising a computing device (20), including a mobile telephone or a tablet, located in the proximity of the container (1), during transport, said computing device (20) comprising positioning means operatively connected to the server (21), to allow tracking positioning data of the container (1) during transport.

11. A method for the controlled transport of biological samples in a container, wherein said container (1) comprises:
- a main body (3) defining a housing cavity;
- a cover (2);
- an opening/closure control device for controlling the opening/closure of the container (1);
- at least one temperature sensor providing information about the internal temperature of the container (1) from an instant of loading the biological samples in the container (1) to a subsequent instant of opening the container (1) and/or extracting the biological samples from the container (1); and
an electronic board configured for collecting the data acquired by at least said temperature sensor and for controlling a state and operation of said opening/closure control device;
the method being **characterized by** the performance of the following steps:
detecting, by means of a content detector controlled by said electronic board, the existence of a biological sample load in the container (1);
sending to a server (21), by means of a communication module included in the electronic board, a series of data about the internal temperature of the container (1), the content and incidences of opening/closing the container (1) and/or incidences related with content handling; and
storing said series of received data and time instants of said incidences of opening/closing the container (1) in a database of the server (21),
wherein said data stored in said database constitutes a control and/or supervision record.

12. The method according to claim 11, further comprising emitting a warning signal if the content detector continues to detect content after extracting the biological samples from the container (1).

13. The method according to claim 11, further comprising emitting a warning signal if the temperature of the container (1) is the same as or exceeds a pre-established threshold temperature value during transport.

14. The method according to claim 11, 12 or 13, wherein said sending of the series of data is performed every certain configurable period of time.

15. the method according to any one of claims 11 to 14, further comprising tracking positioning data of the container (1) from an instant of closing to an instant of opening the container, during transport, using positioning means of a computing device (20) operatively connected to the server (21).
